# EUROPEAN PATENT SPECIFICATION

(11) **EP 2 766 068 B1**
(45) Date of publication and mention of the grant of the patent: **07.12.2016**
(21) Application number: 12753116.8
(22) Date of filing: 28.08.2012
(51) Int. Cl.: A61M 5/158, A61M 5/172, A61M 5/142, A61B 5/145

(54) **MEDICAL APPARATUS HAVING INFUSION AND DETECTION CAPABILITIES**
MEDIZINISCHER APPARAT MIT INFUSIONS- UND DETEKTIONSFÄHIGKEITEN
APPAREIL MÉDICAL AYANT DES CAPACITÉS DE PERFUSION ET DE DÉTECTION

(30) Priority: 11.10.2011 GB 201117539; 11.10.2011 US 201161545777 P
(43) Date of publication of application: 20.08.2014
(73) Proprietor: Medizinische Universität Graz, 8036 Graz (AT)
(72) Inventor: REGITTNIG, Werner, 8010 Graz (AT)
(74) Representative: Dilg, Haeusler, Schindelmann Patentanwaltsgesellschaft mbH
(86) International application number: PCT/EP2012/066703
(87) International publication number: WO 2013/053535

(56) References cited:
- WO-A1-2011/091061
- WO-A2-2008/037316
- US-A1- 2006 253 085
- US-A1- 2010 256 593

## Description

The present invention relates to a medical apparatus for supplying a medication fluid (which may comprise a liquid substance with solid and/or gaseous substances contained or dissolved in it) into an organism and for detecting a substance of the organism and to a method for supplying a medication fluid into an organism and for detecting a substance of the organism, wherein in particular the medication fluid comprises insulin and the substance comprises glucose.

### BACKGROUND ART

WO 2009/032588 A1 discloses a combined sensor and infusion set using separated sites, wherein an infusion portion is coupled to a first piercing member and a sensor portion is coupled to a second piercing member, wherein the infusion portion includes a cannula coupled to the piercing member for supplying a fluid to a placement site. The sensor portion includes a sensor coupled to and extending from the base having at least one sensor electrode formed on a substrate and is coupled to the piercing member in a manner that allows the sensor to be inserted at the placement site. Further, this document discloses a system having a single lumen tube with a protruding sensor and an internal needle.

However, it has been observed that a medical system combined of an infusion section and a sensor section may be difficult to manufacture and may be difficult to handle.

What is needed is a medical apparatus that combines the function of an infusion capability and the function of detecting a substance of the organism, wherein the medical apparatus is easy to manufacture, is reliable and has a more simple construction. Further, the medical apparatus should be easy to handle for the patient suffering from diabetes, in particular type I diabetes.

A medical apparatus according to the preamble of claim 1 is known from WO 2011/091061 A1.

### SUMMARY OF THE INVENTION

The invention is defined in claim 1. According to an embodiment of the present invention, a medical apparatus for supplying (or feeding or conveying or introducing or infusing) a medication fluid (being or comprising in particular a liquid material or a substance to be supplied, in particular a watery solution, in particular comprising a medication) into an organism (in particular a human being or an animal) and for detecting (in particular comprising sensing using one or more electrodes) a substance (in particular in solution of a liquid of the organism) of the organism is provided. Thereby, the medical apparatus comprises a casing (in particular manufactured from a biocompatible polymer, the casing providing a base structure for applying and/or placing and/or attaching the medical apparatus at a human being for infusion purpose and simultaneously detection purpose) having a chamber (being in particular as small as possible, in particular providing a volume of 0.1 micro liter to 15 micro liter, in particular between 2 micro liter and 10 micro liter) for accommodating (at least temporarily) the medication fluid (which may in particular flow through the chamber during the infusion process) and having an opening (providing access to the chamber, the opening having any shape and having a cross-sectional dimension such that the insertion needle can be penetrated through the opening) in fluid communication with the chamber (such that the opening provides an access to the chamber from outside the casing). The medical apparatus further comprises a cannula (being in particular of a tubular shape, being in particular manufactured from a flexible material such as plastic, the cannula in particular providing a flexible tube or tubular structure having in particular an outer mantel having a circular or oval cross-sectional shape and enclosing the lumen) having a lumen (providing a space through which the medication fluid can be transported) being in fluid communication with the chamber (such that medication fluid introduced into the chamber may be guided via the lumen within the cannula to an end of the cannula, which is placed within the organism during the infusion process). Further, the medical apparatus comprises an insertion needle (in particular being manufactured from a metal, wherein the insertion needle may have a tip end portion providing a sharp tip for penetrating skin of the human being, the insertion needle being in particular hollow or being of a C-shape) having a receptacle (or a receiving section, which may be formed by an insertion needle lumen, when the insertion needle is hollow or being provided for example by a recess), wherein at least a portion of the insertion needle is removably arrangeable (on particular such that the insertion needle may be withdrawn or removed from the lumen of the cannula by shifting the insertion needle along a longitudinal axis of the insertion needle, wherein during this shifting, the insertion needle is moved through the opening of the casing to an outside of the casing) within the lumen of the cannula (in particular such that the outer wall of the cannula surrounds the insertion needle in the at least a portion of the insertion needle in an annular fashion). The medical apparatus further comprises a sensor system (in particular comprising a detection portion for detecting the substance, and a supply portion or a cable portion for conducting sensor signals indicative of the substance and/or for supplying the sensor system with electric energy and/or a control signal) for detecting the substance of the organism (wherein in particular the detection portion comprises two or three electrodes formed for example by two or three wires that are insulated from each other by one or more isolating layers, wherein in particular a main wire providing a first electrode is surrounded by a second wire by turning the second wire around the first wire, in particular plural times), wherein the receptacle of the insertion needle is configured to removably at least partially receive the sensor system (wherein in particular the receptacle is configured such that the at least partially received sensor system can be removed from the receptacle by withdrawing the insertion needle away from the casing through the opening, wherein in particular the sensor system may be fixed to the casing such that the sensor system remains at a fixed position relative to the casing, when the insertion needle is withdrawn, wherein in particular the sensor system may be in contact with the substance of the organism, when the insertion needle is withdrawn, such that the sensor system is removed from the receptacle of the insertion needle such that the detection portion of the sensor system may directly or indirectly contact the substance of the organism. Further, the medical apparatus comprises a seal (in particular comprising a deformable material, in particular comprising a flexible material, in particular located at the opening or close to the opening, wherein the seal is adapted to annularly (having in particular an oval shape, a circular shape or any other shape forming a ring) sealingly (in particular tightly being in direct contact with an outer surface of the insertion needle such that fluid is prohibited from passing between the seal and the outer surface of the insertion needle) surround the insertion needle, when the insertion needle is arranged in the opening, wherein the seal is adapted to annularly (having in particular an oval shape, a circular shape or any other shape forming a ring) sealingly (in particular tightly being in direct contact with an outer surface of the portion of the sensor system such that fluid is prohibited from passing between the seal and the outer surface of the sensor system) surround a portion of the sensor system, when the insertion needle is removed from the opening and the portion of the sensor system is arranged in the opening.

According to the present invention the seal is adapted to seal the chamber from fluid communication via the opening (to an outside of the casing), when the insertion needle is arranged in the opening (which may be in particular the case, when the medical apparatus is applied or set at the organism for bringing the medical apparatus in position for infusing the medication fluid and detecting the substance), wherein the seal is adapted to seal the chamber from fluid communication via the opening (to an outside of the casing), when the insertion needle is removed from the opening and a portion of the sensor system is arranged in the opening (wherein in particular the at least partially received sensor system in the receptacle is removed from the receptacle and the insertion needle is withdrawn from the opening such that the portion of the sensor system previously arranged within the insertion needle or the receptacle of the insertion needle is now arranged within the opening without being surrounded or at least partially enclosed by the insertion needle.

Thus, the seal has a double function of providing a seal for the chamber in the process of inserting an insertion assembly comprised of the cannula, the insertion needle and the sensor system into the organism and to provide a seal of the chamber, when the insertion needle has been withdrawn to provide an infusing/detection assembly, which is comprised of the cannula and the sensor system without the insertion needle, wherein a detection portion of the sensor system is arranged to be in contact with the substance of the organism and an exit port of the cannula is arranged within the organism (in particular below the skin surface) to enable supplying the medication fluid into the organism.

Thereby, only one seal is required to seal the chamber from communication via the opening and to provide feed through of a cable section to the detection portion of the sensor system. Thereby, the construction of the medical apparatus may be simplified. In particular, sealing of the chamber may be simplified and reliably ensured. Thereby, during the insertion process, where the insertion needle is still present within the cannula and within the opening, the insertion needle sticks through the opening, while the seal seals the chamber from fluid communication via the opening. The insertion assembly with the insertion needle sticked through the opening sealed by the seal is then inserted into the organism, in particular comprising penetrating the insertion needle, in particular a tip end portion of the insertion needle, through the skin of the human being, while the insertion needle may not move relative to the casing and may not move relative to the opening and the seal.

When the insertion needle harboring at least partially the sensor system and the cannula at least partially surrounding the insertion needle have completely been inserted into the organism, wherein the casing is in particular placed at an outer surface of the organism, in particular outside the skin surface of the organism, the insertion needle may be withdrawn from the organism by moving the insertion needle relative to the casing and relative to the cannula and relative to the sensor system to an outside of the casing, in particular by shifting the insertion needle along a longitudinal axis of the insertion needle.

Thereby, the insertion needle is moved through the opening, while the seal seals the chamber from fluid communication via the opening. Thereby, it is avoided that contaminants can reach the chamber or that medication fluid already present within the chamber leaves the chamber to an outside of the casing. The insertion needle is continued to be withdrawn from the casing through the opening until the tip end portion of the insertion needle has been run through or moved through the opening. When the insertion needle has passed the opening completely, the portion of the sensor system may be, within the opening, surrounded by the seal, since the portion of the sensor system has not moved while withdrawing or moving the insertion needle through the opening. Thus, the portion of the sensor system remains at a same position within the opening relative to the casing while withdrawing the insertion needle.

When the insertion needle has been moved through or run through the opening, so that the tip end portion of the insertion needle has left the opening, the seal may deform to contact the portion of the sensor system still present within the opening. In particular, the seal will surround the portion of the sensor system in an annular manner to be in contact with the portion of the sensor system as well as being in contact with a delimiting wall section of the casing delimiting the opening.

Thereby, a reliable seal function is enabled during the insertion process as well as during the process of withdrawing the insertion needle as well as during the process of supplying the medication fluid into the organism and detecting the substance of the organism. Thereby, a reliable medical apparatus may be provided, which may be easy to manufacture and may be easy to handle for the patient.

The medical apparatus may in particular be applied or used by patients, which suffer from type I diabetes, in which their own production of insulin is impaired. These patients have to supply insulin from outside in order to avoid dangerous situations of high blood glucose concentration. In particular, patients suffering from type I diabetes may have to supply insulin at plural instances everyday. For this purpose, the medical apparatus may be supplied with an insulin pump, which enables supply of insulin into the organism on demand, such as stepwise or in a continuous fashion.

The medical apparatus is further applicable or may be used for or from patients, which suffer from type II diabetes.

Further, the medical apparatus may be adapted to adjust the supplied amount of medication fluid, in particular insulin, according to or based on the current concentration of glucose within the blood. In particular, the medical apparatus may estimate or determine the concentration of glucose within the blood from the detected substance, which may represent a concentration value in a particular tissue of the organism, in particular fat tissue. In particular, also the medication fluid, in particular insulin, may be supplied to fat tissue of the patient taking the determined glucose level into account. Thereby, insulin administrations at appropriate dosages and timing may be provided which in turn may lead to an accurate or tight control of the patient's blood glucose concentration.

The sensor system may in particular comprise a glucose sensor, which is enabled to measure the glucose concentration, when inserted in fat tissue, in particular below the skin of the abdomen. In particular, the sensor system may be adapted to measure glucose concentration continuously or in a stepwise manner.

In particular, the insulin pump may supply insulin to the chamber using a tube being connected to the casing either at the opening or at a further opening in fluid communication with the chamber.

In particular, the medical apparatus may be adapted to be placed at the skin of the patient for infusing insulin and detecting glucose during a period of time between one and five days. After this period of time, the medical apparatus may be removed from the patient and a new medical apparatus may be applied to another location at the skin of the patient, in order to avoid infections or other medical problems.

The medical apparatus may further be adapted to transmit sensor data to the insulin pump in a wire-based or wireless manner.

Advantageously, for supplying the medication fluid into the organism and detecting the substance of the organism, only one piercing location is required.

Experiments have been conducted, wherein insulin was supplied to a particular fat tissue location and tissue fluid was taken from this location to measure the glucose concentration. It could be shown that in spite of varying supply of insulin, a stable ratio was established between the glucose concentration within the blood and the glucose concentration in the tissue fluid at the location, where the insulin was supplied. Thus, from the measured glucose concentration obtained by the sensor system, the concentration of the glucose within the blood can be derived, for example using one or more calibration curves or tables.

The sensor system may comprise a detection portion, which may be sensitive to the substance to be detected. This sensor portion may either be arranged within the lumen of the cannula, wherein in particular the cannula is a perforated cannula. Alternatively or additionally, the detection portion may protrude beyond the end of the cannula protruding further into the tissue of the organism.

Before, during or after placing the medical apparatus at the patient (involving inserting the insertion needle into the tissue of the organism), the cannula may be connected via the chamber to the medication fluid tube and the sensor may be connected to a transmitter or to a cable for controlling the sensor and/or receiving the sensor signals.

The medical apparatus according to the embodiment of the present invention combines the glucose sensor within the infusion cannula in a very simple manner such that only slight modifications are required at conventional infusion cannula casings and sensor holders. Thereby, manufacturing costs may be reduced. Further, the medical apparatus may be dimensioned to be relatively small (such that for example the casing may have a dimension of 0.5 cm to 2 cm wide and 0.1 cm to 0,5 cm high. Further, the number of stitches or insertion processes of the insertion needle may be reduced, thus reducing discomfort to the patient.

According to an embodiment of the present invention, the seal comprises a deformable (in particular flexible) biocompatible (which does not impair biological functions or structures) material, such as a silicone elastomer, ethylene propylene rubber, polyurethane elastomer and/or other suitable elastomeric materials. In particular, a silicone elastomer may be easily manufactured in a cost-effective manner, to reduce the costs of the medical apparatus. Further, silicone elastomers are well-known substances being applied in a number of bio-medical applications and being known to be biocompatible. Thereby, the safety of the medical apparatus may be improved. Thereby, medical risks may be reduced or even avoided.

According to an embodiment of the present invention, the seal surrounds (or encloses) the insertion needle (in particular at least a portion of the insertion needle) at the opening in an annular manner, when the insertion needle is arranged in the opening (in particular during inserting the insertion assembly (comprising the insertion needle within the cannula and the portion of the sensor system within the insertion needle) and during the process of withdrawing the insertion needle from the casing.

Further, the seal surrounds the portion of the sensor system in an annular manner, when the portion of the sensor system is arranged in the opening, but the insertion needle is not arranged in the opening, which is the case when the insertion needle is completely withdrawn from the casing to provide the infusion/detection assembly, which enables administration of the fluid into the organism and detection of the substance of the organism. By surrounding the insertion needle or the portion of the sensor system in the different configurations or operational states of the medical apparatus, the sealing function may be reliably ensured. Further, the insertion needle may be easily guided through the opening, while the seal surrounds the insertion needle.

According to an embodiment of the present invention, the portion of the sensor system arranged in the opening comprises a sensor cable (including in particular two, three or more wires leading to separate sensor electrodes) for leading a sensor signal through the opening to an outside of the chamber (and/or for leading a control signal from the outside to the detection portion). Thereby, a sensor signal indicative of the substance of the organism, in particular being indicative of a concentration of the substance of the organism, can be communicated from the detection portion of the sensor system through the opening via the sensor cable and can be supplied to a processing system or display system for processing or displaying the sensor signal.

According to an embodiment of the present invention, the cannula is fixed at the casing by an adhesive or by using a bushing. Having the cannula fixed at the casing avoids that the cannula is moved relative to the casing in particular when the insertion needle is withdrawn from the casing after having inserted the insertion assembly comprising the cannula, the insertion needle within the cannula and the sensor portion within the insertion needle into the organism. Thereby, it may be ensured that the cannula is maintained at the intended position within the organism, when the insertion needle is withdrawn. Further, it is enabled to fix the cannula within the organism by fixing the casing at the surface of the organism, in particular at the skin of the organism, for example by using a double adhesive band or any other adhesive.

According to an embodiment of the present invention, the cannula is flexible (in particular manufactured of a polymer) and comprises an insertion portion (representing the portion of the cannula, which is intended to be inserted into the organism such that the insertion portion is completely below the surface of the organism), protruding from the casing away from the chamber. In particular, the insertion portion may protrude from the casing perpendicular or transverse to a surface of the casing being intended to be placed onto the skin of the human being. At an end of the insertion portion, which may represent a section of the cannula inserted deepest into the organism, the cannula may have an opening or an exit port for supplying the medication fluid into the organism.

According to an embodiment of the present invention, the sensor system comprises a detection portion protruding beyond the insertion portion (in particular beyond the end of the insertion portion or the exit port of the cannula) of the cannula away from the chamber, wherein the detection portion is configured to detect the substance. The detection portion in particular may comprise two or more wires, which are insulated from each other by one or more layers, and wherein the two or more wires represent two or more sensor electrodes. One wire may be wound around the other wire at least partially.

The sensor portion may be an electrochemical sensor portion, which may be responsive to a concentration of the substance. Upon being in contact with the substance within the organism, the detection portion may generate a (electrical) sensor signal indicative of the concentration of the substance. In particular, the detection portion may be inserted into the organism farther than the end portion or exit port of the cannula. Thereby, the substance within the organism may easily contact the detection portion, since the detection portion is advantageously exposed and surrounded by the tissue of the organism such that the substance in the tissue of the organism can directly get in contact with the detection portion. Thereby, a measuring accuracy of the sensor system may be improved.

According to an embodiment of the present invention, the insertion portion of the cannula comprises at least one entry port (or two entry ports and three, four, five, six or 2-100 entry ports) at an outer surface (in particular a cylindrical surface) of the insertion portion of the cannula, wherein the entry port allows entry of the substance into the lumen of the cannula (and wherein in particular the entry port also allows passing the medication fluid from the lumen of the cannula to an outside of the cannula thus into the tissue of the organism). Thereby, the insertion portion is in particular perforated having a plurality of holes for fluid communication between the lumen of the cannula and the outside of the cannula. Thereby, the sensor system comprises a detection portion arranged within the insertion portion of the cannula (but not protruding beyond the insertion portion of the cannula), wherein the detection portion is configured to detect the substance entered into the lumen of the cannula, in particular via the at least one entry port or via the perforated wall of the cannula. Thereby, the sensor portion is protected from mechanical damage when being inserted into the organism. Further, the substance may easily diffuse through the at least one entry port or through the perforated cannula wall in order to get in contact with the detection portion of the detection or sensor system. Thereby, a reliable sensor system may be provided. Moreover, a pumping direction of the pump which has previously pumped the medication fluid into the body may be simply reversed so that the substance surrounding the cannula wall is sucked through the at least one entry port or through the perforations of the cannula wall into the cannula lumen. Thereby, the speed with which the substance is transported from the tissue to the detection portion of the detection or sensor system may be increased.

According to an embodiment of the present invention, an insertion assembly comprising at least a portion of each of the cannula, the insertion needle and the sensor system is insertable into the organism (in particular as a whole into the organism using the casing or another particular placing facility), wherein the insertion needle is at least partially arranged within the lumen of the cannula and the sensor system is partially received in the receptacle of the insertion needle. During the insertion of the assertion assembly, the cannula, the insertion needle and the sensor system may be at fixed positions relative to each other, such that these elements (the cannula, the insertion needle and the sensor system) do not move relative to each other during the insertion process. Thereby, mechanical damage of any of the elements may be avoided during the insertion process.

According to an embodiment of the present invention, in the insertion assembly, the insertion needle is located at least partially within the lumen of the cannula such that a tip end portion of the insertion needle protrudes beyond a longitudinal end (or exit port) of the cannula farthest away from the chamber. In particular, the longitudinal end of the cannula has the opening or exit port for supplying the medication fluid into the organism.

The insertion needle, in particular the tip end of the insertion needle, protrudes beyond the opening of the cannula to facilitate the insertion process, since the tip end portion of the insertion needle may easily be pierced through the skin of the organism and may easily be penetrated into the organism. In particular, the opening at the longitudinal end of the cannula may be formed by an annular wall section of the cannula tightly fitting around the outer surface of the insertion needle, such that the insertion needle and the cannula are press-fitted to each other and/or such that an outer dimension of the insertion needle matches an inner dimension of the cannula.

In particular, the longitudinal end of the cannula may comprise a conically shaped cylindrical wall section, wherein the conical angle may match an angle of the tip end portion of the insertion needle to provide a smooth transition of the surface provided by the tip end portion of the insertion needle and the surface portion provided by the outer surface of the cannula at the longitudinal end of the cannula. Thereby, the insertion process may be facilitated without causing discomfort for the patient.

According to an embodiment of the present invention, the insertion assembly is insertable, along a longitudinal direction of the insertion needle, into the organism covered by skin, wherein a skin surface is transverse, in particular including an angle between 20° and 90°, in particular perpendicular, to the longitudinal direction of the insertion needle. Thereby, depending on the application, in particular the tissue or the patient, different insertion directions relative to the skin of the patient are supported by the medical apparatus according to embodiments of the present invention. Thereby, the medical apparatus may be used in a variety of applications.

According to an embodiment of the present invention, the medical apparatus further comprises a placement facility having a body and a lever, the lever being removable connectable with the casing, wherein moving the lever enables moving the casing in a direction along the longitudinal axis of the insertion needle relative to a body of the placement facility for inserting the insertion assembly into the organism.

Thereby, the placement facility may comprise a flexible element, such as a spring element, in order to move the casing relative to the body of the placement facility using the spring in particular being connected to the lever. Thereby, the patient does not need to insert the insertion assembly by hand into the tissue but may apply the placement facility, connect the lever to the casing and have the placement facility introduce the insertion assembly, until the casing of the medical apparatus contacts the skin of the patient, in particular having an adhesive tape placed in-between.

According to other embodiments of the present invention, the medical apparatus comprises a handle portion connectable to the casing, in particular during or before the insertion process, to enable a more simple handling of the insertion assembly connected to the casing. The handle portion may comprise a section for allowing a hand of the patient to grip the handle portion and thus to navigate the casing (connected to the handle portion) with the insertion facility to the insertion position and to actually enable inserting the insertion assembly into the organism.

According to an embodiment of the present invention, the medication fluid guided inside the lumen of the cannula is in contact with a detection portion or another portion of the sensor system, when the medication fluid is supplied into the organism. Thereby, the construction of the medical apparatus may be simplified.

According to an embodiment of the present invention, the cannula has a tubular shape, wherein in particular the lumen of the cannula is enclosed in a cylindrical wall, having a cross-sectional inner extent, which matches a cross-sectional outer extent of the insertion needle. Thus, in the insertion assembly the cannula and the insertion needle may have dimensions such that the insertion needle may be tightly contacting an inner surface of the cannula, when the insertion assembly is formed. Nevertheless, the insertion needle may be withdrawn from the lumen of the cannula without damaging or even destroying the cannula. By the matching, the cross-sectional inner extent of the cannula with the cross-sectional outer extent of the insertion needle, it may be avoided that tissue of the organism is introduced into the cannula during the insertion process. In particular, there may be substantially no gap between the outer surface of the insertion needle and the inner surface of the cannula at least at the longitudinal end of the cannula from which the tip end portion of the insertion needle may protrude, such as for example 0.1 mm to 2 mm.

According to an embodiment of the present invention, the insertion needle has a C-shape (at least in a portion thereof, wherein the insertion needle may have for example a cross-sectional shape of a half-circle or in general a portion of a circle) or has a tubular shape (thus also providing an insertion needle lumen, in particular delimited by a cylindrical insertion needle wall).

If the insertion needle has a tubular shape, the insertion needle may be a conventional hollow needle, which may reduce the manufacturing costs. Providing the insertion needle with a C-shape, allows withdrawing the insertion needle even if the sensor system comprises a kink. In particular, the kink of the sensor system may be provided at a cable portion of the sensor system and the cable portion may be secured at a position at the casing to form for example one or more electrode terminals of the sensor system. These sensor terminals may be preformed, i.e. formed before the medical apparatus is inserted into the organism. Thereby, it may be avoided to contact a cable portion of the sensor system after having inserted the insertion assembly into the organism. Thereby, handling of the medical apparatus may be simplified.

According to an embodiment of the present invention, the casing has a further opening in fluid communication with the chamber, wherein a connector for connecting a medication fluid supply tube, is provided at the further opening. In particular the medication fluid supply tube may comprise a pipe having a tube lumen. Thereby, it is enabled to supply, via the (in particular lumen of the) medication fluid supply tube, medication fluid into the chamber, from which it may be supplied via the cannula to the inside of the organism.

According to an embodiment of the present invention, the medical apparatus further comprises an adapter having at least one electrical terminal, wherein the adapter is releasably connectable to the casing and wherein the electrical terminal is electrically contactable to a sensor cable of the sensor system. The adapter may in particular be adapted to contact one or more wires of the sensor cable and provide electrical connections of these one or more wires to electrical terminals, which may form one or more sockets, to which respective plugs may be connected, when the insertion assembly is completely inserted into the organism. This adapter may in particular be provided, when the insertion needle has a tubular shape such that the sensor cable may not provide any sockets to which electrical circuitry can be connected via corresponding plugs.

According to an embodiment of the present invention, the adapter comprises a transmitter for transmitting sensor signals received from the sensor system, in particular to a processing or control system, which may be connected to a pumping system for controlling the supply of the medication fluid in dependence of the received sensor signals. The transmitter may wirelessly transmit the sensor signals, e.g. using radio frequency electromagnetic waves, electromagnetic inductance or any other wireless communication technique. Thereby, having placed the medical apparatus at the skin of the patient, any electrical wires leading away from the medical apparatus may be avoided for simplifying the handling of the medical apparatus and for avoiding discomfort of the patient.

According to an embodiment of the present invention, the sensor system comprises a kink, in particular at the opening or at a location farther away from the chamber than the opening. Providing a kink at the sensor system may enable to branch-off the sensor system from the longitudinal direction of the insertion needle such that the insertion needle does not need to be withdrawn from the entire sensor system, but only from a portion of the sensor system. In particular, the kink of the sensor system may be in a cable portion of the sensor system and wires of the cable portion of the sensor system may be fixed at the casing and/or connected to sockets, to which corresponding plugs may be electrically connected. Thereby, connecting the sensor system may be simplified.

According to an embodiment of the present invention, the portion of the sensor system runs at least through the seal along the longitudinal axis of the insertion needle. Thereby, only one opening is required to pass the combination of the insertion needle and the sensor portion through the opening in a same direction, thus simplifying the contruction.

According to an embodiment of the present invention, the sensor is at least partially guided within the insertion needle within the cannula through the seal during inserting the insertion assembly into the tissue by moving the insertion needle into the tissue. Thereby, in particular, the sensor system, the insertion needle and the cannula may be at fixed positions relative to each other during the insertion process.

According to an embodiment of the present invention, the insertion needle is hollow forming a needle lumen, wherein the needle lumen serves as the receptacle. Thereby, the insertion needle may be easy manufactured and may be a conventional insertion needle being selected to have the appropriate inner dimension and outer dimension. In particular, the insertion needle is selected such that the sensor or at least a sensor portion may be placed within the needle lumen and the insertion needle is further selected such that the outer surface of the insertion needle fits into the lumen of the cannula.

According to an embodiment of the present invention, the casing has a connector connecting a medication fluid tube for supplying medication fluid to the chamber. The connector may be provided at a further opening or the connector may be provided at the seal and/or arranged within the seal at the opening. Thereby, supply of the medication fluid to the chamber may be achieved via the medication fluid tube, which is connected to the connector. Arranging the connector within the seal or at the seal avoids the requirement for a further opening for supplying the medication fluid into the chamber. Thereby, the medical apparatus may be even more simplified.

According to an embodiment of the present invention, the medical apparatus further comprises a pump system connectable to the medication fluid tube in fluid communication with the chamber for supplying the medication fluid into the chamber and from there via the cannula into the organism. Thereby, the pump system may allow adjusting a particular flow rate of the medication fluid such that a defined amount of medication fluid per time interval may be supplied into the organism. Thereby, the medical apparatus may be adapted to supply a defined amount of medication fluid to the organism per time interval.

According to an embodiment of the present invention, the medical apparatus further comprises a controller communicatively, in particular electrically, connected to the sensor system and communicatively, in particular electrically, connected to the pump system, wherein the controller is adapted to control the pump system based on a signal received from the sensor system. Thereby, it is enabled to adjust a flow rate of the medication fluid or an amount of the medication fluid supplied to the organism per time interval to vary when the substance detected by the sensor system varies. For example, when it is determined by the sensor system that the substance exhibits an increasing concentration, the amount of medication fluid supplied per time interval into the organism may be increased or decreased depending on the particular substance detected and medication fluid supplied. For example, when increasing or decreasing glucose concentration is detected by the sensor system, the flow rate or amount of insulin supplied to the organism may be increased or decreased, respectively.

According to an embodiment of the present invention, the casing is releasably connectable at the organism, in particular at a skin of the organism, in particular using an adhesive force. Thereby, the insertion assembly from which the insertion needle has been removed may be secured at the desired administration position, since the cannula having the sensor portion therein is fixed at the casing. Thereby, the cannula and the sensor system may be secured to a particular location within the organism.

According to an embodiment of the present invention, the substance comprises glucose and the medication fluid comprises insulin. In particular, the medical apparatus may be used from a patient suffering from type I diabetes, wherein the medical apparatus may be carried by the patient for 1 to 5 days and may then be replaced with a new medical apparatus according to an embodiment of the present invention.

It should be understood that features (individually or in any combination) disclosed, described, explained or employed for a medical apparatus for supplying a medication fluid into an organism and for detecting a substance of the organism, may also be applied, used for or employed (individually or in any combination) with a method for supplying a medication fluid into an organism and for detecting a substance of the organism according to an embodiment of the present invention and vice versa.

According to an embodiment of the present invention, it is provided a method for supplying a medication fluid into an organism and for detecting a substance of the organism, the method comprising: providing an insertion needle, having a receptacle, in an opening being in fluid communication with a chamber of a casing, the chamber being provided for accommodating the medication fluid, wherein a sensor system is at least partially received in the receptacle; wherein at least a portion of the insertion needle is removably arranged within a lumen of a cannula, wherein the lumen of the cannula is in fluid communication with the chamber; annularly sealingly surrounding, by the seal, the insertion needle when the insertion needle is arranged in the opening (in particular sealing, using a seal, the chamber from fluid communication via the opening when the insertion needle is arranged in the opening); removing the insertion needle from the opening; annularly sealingly surrounding, by the seal, a portion of the sensor system when the insertion needle is removed from the opening and the portion of the sensor system is arranged in the opening (in particular sealing the chamber from fluid communication via the opening when a portion of the sensor system is arranged in the opening); detecting the substance of the organism using the sensor system; and supplying the medication fluid into an organism via the cannula.

Embodiments of the present invention are now described with reference to the accompanying drawings. The invention is not limited to the illustrated or described embodiments. Similar elements may be denoted by similar reference sign differing only in the first digit. Reference signs in the claims are not delimiting.

### BRIEF DESCRIPTION OF THE DRAWINGS

- Fig. 1A: schematically illustrates a side view of a medical apparatus according to an embodiment of the present invention having an insertion assembly attached;
- Fig. 1B: schematically illustrates a side view of a medical apparatus according to an embodiment of the present invention having a transmitter attached and a tube connected;
- Fig. 1C: schematically illustrates a cross-sectional side view of a portion of the medical apparatus and the insertion assembly shown in Fig. 1A,;
- Fig. 1D: schematically illustrates a cross-sectional side view of a portion of the medical apparatus illustrated in Fig. 1B;
- Fig. 2A: schematically illustrates a side view of a medical apparatus according to another embodiment of the present invention having an insertion assembly attached;
- Fig. 2B: schematically illustrates a side view of the medical apparatus illustrated in Fig. 2A having the insertion needle withdrawn and having a tube connected;
- Fig. 2C: schematically illustrates a cross-sectional top view of the medical apparatus illustrated in Fig. 2B;
- Figs. 3A, 3B 3C, 3D: schematically illustrate cross-sectional side views of medical apparatuses according to further embodiments of the present invention;
- Figs. 4A and 4B: schematically illustrate portions of a medical apparatus according to embodiments of the present invention, in particular illustrating details of the sensor and the cannula;
- Fig. 5: schematically illustrates a perspective view of a medical apparatus according to an embodiment of the present invention having a handle portion attached for inserting the medical apparatus into the organism; and
- Figs. 6A and 6B: schematically illustrate perspective top views of medical apparatuses according to embodiments of the present invention attached to a skin of a patient and having a medication fluid tube connected.

### DETAILLED DESCRIPTION OF EXEMPLARY EMBODIMENTS

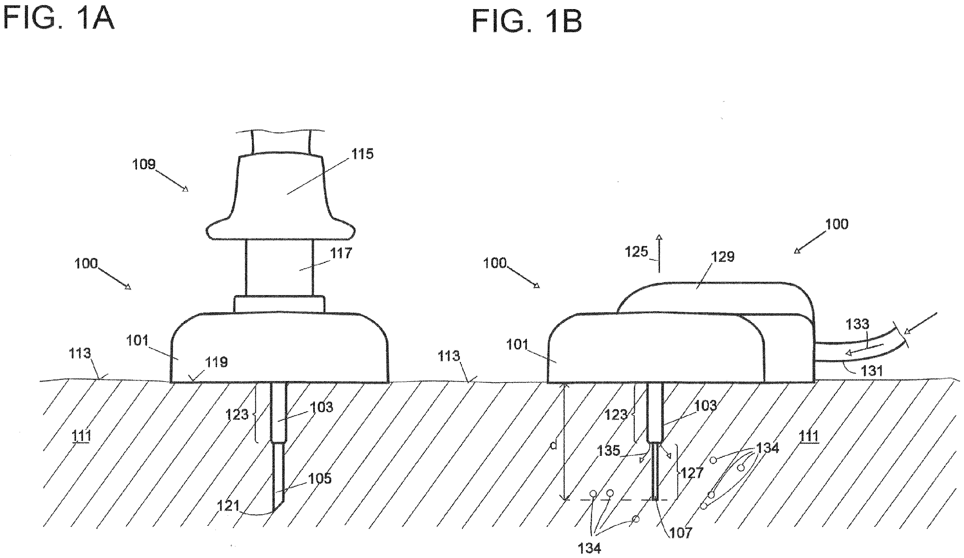

Fig. 1A schematically illustrates a side view of a medical apparatus 100 according to an embodiment of the present invention, wherein the medical apparatus comprises a casing 101, a cannula 103, an insertion needle 105 and a not illustrated sensor within the insertion needle 105, which is however illustrated in Fig. 1B as the sensor 107. The medical apparatus 100 further comprises a handle portion 109, which is releasably connected to the casing 101. The handle portion 109 may be gripped by hand for inserting the medical apparatus 100 into the tissue 111, which is covered by skin 113.

In particular, in Fig. 1A a situation is illustrated, in which the medical apparatus 100 has just been placed at the skin 113 of the patient and wherein the cannula 103, the insertion needle 105 arranged in the lumen of the cannula 103 and the sensor system being at least partially received in a receptacle of the insertion needle 105 (these three elements forming an insertion assembly) have been inserted into the tissue 111 of the patient.

The handle or gripping portion 109 comprises a handle 115 and a connection portion 117 for connecting the gripping portion 109 to the casing 101. For this purpose, the connection portion 117 and the casing may comprise a releasable locking mechanism, such as a snap mechanism.

The casing 101 comprises a patient contacting surface 119, which may be brought via an adhesive or an adhesive band in contact with the skin 113 of the patient. Thereby, the casing 101 is fixed at the skin 113 of the patient such that the cannula 103, the insertion needle 105 and the sensor 107 within the insertion needle 105 are secured at a particular insertion site within the tissue 111.

The insertion needle 105 further comprises a tip end portion 121, which provides a sharp tip for piercing through the skin 113 and further into the tissue 111, which may in particular comprise fat tissue. The cannula 103 is inserted along an insertion portion 123 into the tissue 111. The insertion needle 105 protrudes into the tissue 111 beyond the insertion portion 123, wherein an extent of this protrusion may depend on the application. According to a particular application, the tip end portion 121 of the insertion needle 105 just slightly protrudes beyond the insertion portion 123 of the cannula 103 such that only the tip end portion 121 protrudes beyond the insertion portion 123 of the cannula 103.

Fig. 1B illustrates the medical apparatus 100 illustrated in Fig. 1A after the insertion needle 105 has been withdrawn in a direction labeled by arrow 125, which in the illustrated embodiment is approximately perpendicular to a surface of the skin 113. In particular, the insertion needle 105 has been withdrawn after the gripping portion 109 has been released from the casing 101 or wherein at least the gripping portion 109 has been unlocked from the casing 101 and has been lifted or moved along the direction 125, while the insertion needle 105 was attached to the gripping portion 109, to move the insertion needle away from the skin together with the gripping portion.

After withdrawing the insertion needle 105, a sensor portion 127 of the sensor 107 is exposed within the tissue 111 to protrude beyond the insertion portion 123 of the cannula 103. Thereby, the detection portion 127 of the sensor system 107 is exposed to the tissue 111 below the skin 113. The depth d to which the sensor portion 127 is inserted into the tissue, may amount to between 1 mm and 20 mm, in particular between 6 mm and 12 mm. A cross-sectional extent of the sensor portion 127 may amount to between 0.1 mm-2 mm and a cross-sectional extent of the cannula 103 may amount to between 0.2 mm-3 mm. The cannula 103 is formed from a flexible material.

The medical apparatus 105 further comprises an adapter 129 attached to and connected to the casing 101, wherein the adapter 129 comprises a transmitter, which is connected to a sensor cable of the sensor system 107 to operate the sensor system 107 and receive sensor signals from the sensor system 107.

Further, the medical apparatus 100 is provided with a medication fluid supply tube 131, which supplies medication fluid 133 into a not illustrated chamber of the casing 101 from which the medication fluid 133 is guided through a lumen of the cannula 103 to be supplied to the tissue 111 as indicated by arrows 135. Thus, the medical apparatus 100 is adapted to simultaneously supply medication fluid 133, such as insulin, to the tissue 111 and detect a substance 134 sketched by circles, in particular glucose, using the sensor portion 127, which is responsive to the substance 134 to be detected.

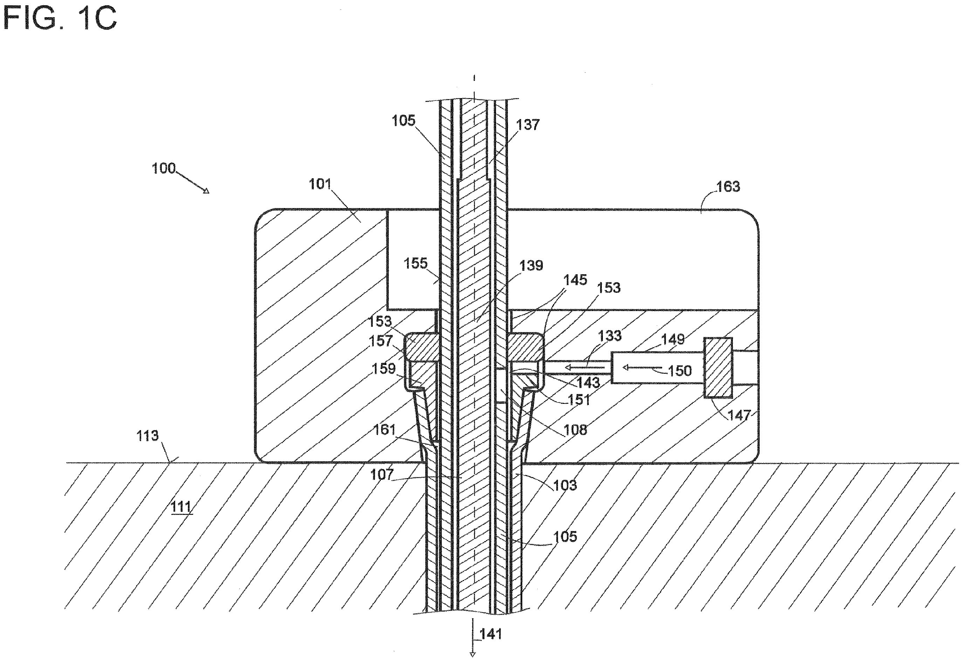

Fig. 1C schematically illustrates a portion of the medical apparatus 100 illustrated in Fig. 1A in a situation, where the medical apparatus still comprises the insertion needle 105 for inserting the insertion needle 105, the sensor portion 127 arranged within the hollow needle 105 and the insertion portion 123 of the cannula 103 into the tissue 111. In particular, an insertion needle lumen 137 serves as a receptacle for receiving the sensor system 107 at least partially. In particular, the portion of the sensor system 107 received within the insertion needle lumen 137 represents a cable portion 139 of the sensor system 107. The cable portion 139 may comprise two or more electrical wires, that lead to electrodes of the detection portion 127 (not illustrated in Fig. 1C) being in contact (directly or indirectly) with the tissue 111.

As can be seen from Fig. 1C, the insertion needle 105 has a longitudinal axis labeled as 141, which is oriented during the insertion process approximately perpendicular to the surface of the skin 113. The insertion needle 105 is hollow to accommodate the cable portion 139 of the sensor system 107.

The medical apparatus 100, in particular the casing 101, comprises a chamber 143 (in particular having a volume between 1 micro liter and 30 micro liter), which is substantially occupied by the insertion needle 105 being penetrated through an opening 145 and the chamber 143 and further within the cannula 103 into the tissue 111. Via a connector 147 the medication fluid supply tube 131 is connected to a supply channel 149 arranged within the casing 101, wherein the supply channel 149 leads via a further opening 151 to the chamber 143. Optionally, an additional opening 108 (as indicated in Fig. 1C) in the wall of the insertion needle 105 can be provided to allow filling the needle lumen 137 with the medication fluid before insertion, thereby ridding the needle lumen 137 and the cannula lumen 161 of air before use.

Fig. 1C illustrates the situation, where the cannula 103, the insertion needle 105 and the sensor portion 127 (representing an insertion assembly) are still to be inserted or have just been inserted into the tissue 111. However, the insertion needle 105 has not yet been withdrawn. To seal the chamber 143 from fluid communication to an outside of the casing 101 via the opening 145, a silicone seal 153 is provided at the opening 145, which surrounds the insertion needle 105 in an annular fashion. Thereby, the seal 153 at the opening 145 contacts an outer surface 155 of the insertion needle 105 and also contacts a casing wall 157 delimiting the opening 145. Thereby, introduction of contaminants from outside the casing 101 into the chamber 143 is avoided. Further, also leakage of the medication fluid 133 present within the chamber 143 via the opening 145 to an outside of the casing 101 is avoided.

Using a bushing 159 having an annular shape, being in particular conically shaped, the cannula 103 is fixed at the casing 101. As can be seen from Fig. 1C, at least a portion of the insertion needle is removably arrangeable within the lumen 161 of the cannula 103.

The medical apparatus 100 further comprises at the casing 101 a receiving area 163 for receiving the adapter 129, as illustrated in Fig. 1B.

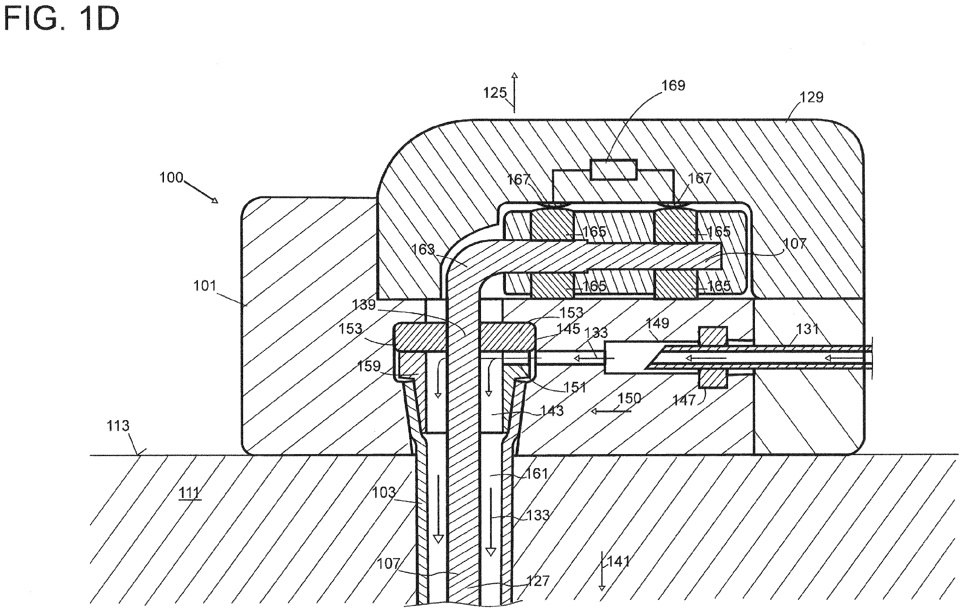

Fig. 1D schematically illustrates the medical apparatus 100 illustrated in Fig. 1C in the situation after having withdrawn the insertion needle 105 from the casing 101, Fig. 1D corresponding to the situation illustrated in Fig. 1B. As can be seen from Fig. 1D, the insertion needle 105 has been withdrawn along the direction labeled with arrow 125, but the cannula 103 and the sensor 107 have maintained their positions. In this situation the medication fluid 133 is introduced via the medication fluid supply tube 131, the connector 147 or seal 147, the supply channel 149 into the chamber 143 within the casing 101 and from there via the lumen 161 of the cannula 103 into the tissue 111.

As can be seen by comparing Figs. 1C and 1D, the silicone seal 153 has deformed and now contacts the cable portion 139 of the sensor system 107 instead of contacting the outer surface 155 of the insertion needle 105 as was the case in the situation illustrated in Fig. 1C. Thereby, the seal 153 seals the chamber 143 from fluid communication via the opening 145 when the insertion needle 105 is removed from the opening 145 and a portion of the sensor system is arranged in the opening 145. Thereby, in particular leakage of the medication fluid 133 being present within the chamber 143 and flowing through the chamber 143 into the lumen 161 of the cannula 103, is avoided.

As can be seen from Fig. 1D, the cable portion 139 of the sensor system 107 comprises a kink 163 in a region above the opening 145 and also above the seal 153, wherein the kink 163 has been achieved by bending the cable portion 139. Not illustrated wires of the cable portion 139 may be electrically connected to contact pads 165, which may be in turn electrically connected to not illustrated socket elements enabling connecting the sensor system electrically using appropriate plug elements.

Alternatively, as illustrated, the contact elements 165 are electrically connected to transmitter contact elements 167, which lead sensor signals to a transmitter 169, which is enabled to wirelessly transmit the sensor signals to a monitoring or processing system not illustrated in Fig. 1D. The control and processing system may in turn be connected to a pump system for controlling a pump for adjusting a supply or a flow rate or an amount of the medication fluid 133 via tube 131, which is then infused into the tissue 111.

As can be seen from Fig. 1C and Fig. 1D, the supply channel 149 is in communication with the chamber 143 via the further opening 151, wherein the further opening is located closer to the surface of the skin 113 than the opening 145. Further, a longitudinal direction 150 of the supply channel 149 is substantially perpendicular to the longitudinal axis 141 of the insertion needle 105 along which the insertion assembly is inserted into the tissue 111.

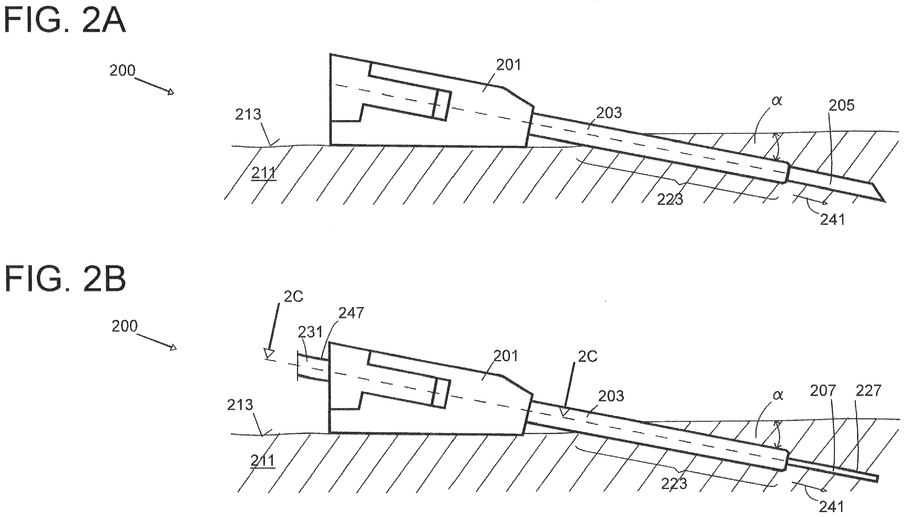

Fig. 2A schematically illustrates a medical apparatus 200 according to another embodiment of the present invention in a schematic side view.

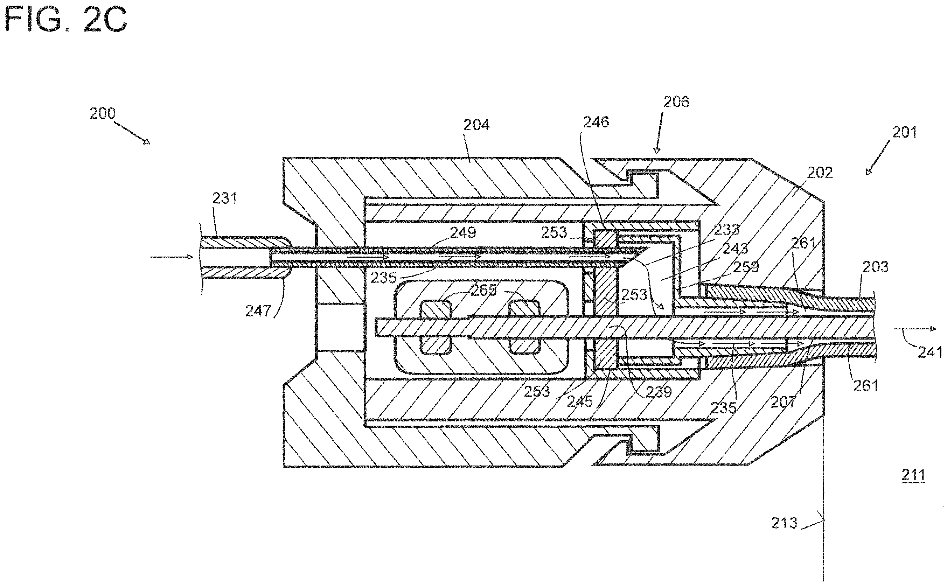

Fig. 2B schematically illustrates a side view of the medical apparatus illustrated in Fig. 2A having the insertion needle withdrawn and having a tube connected. In contrast to the medical apparatus 100 illustrated in Figs. 1A, 1B, 1C and 1D, the medical apparatus 200 illustrated in Figs. 2A, 2B and 2C is inserted at an acute angle α between 10° and 45° (measured as the angle between the longitudinal axis 241 of the insertion needle 205 and the surface of the skin 213).

The medical apparatus 200 also comprises a casing 201, a cannula 203 fixed at the casing 201, an insertion needle 205 being at least partially arranged within the cannula 203 and a sensor portion 227 of a sensor system 207 received at least partially within the insertion needle 205 or at a receptacle of the insertion needle 205.

The medication fluid supply tube 231 is connected to a connector 247, which supplies the medication fluid into the chamber 243 as is depicted in Fig. 2B. Fig. 2C schematically illustrates a cross-sectional top view of the medical apparatus 200 illustrated in Fig. 2B taken along the line labeled 2C in Fig. 2B. As can be seen in Fig. 2C, the medication fluid supply channel 249 is oriented approximately parallel to the longitudinal axis 241 of the insertion needle, which has already been withdrawn in the illustration of Fig. 2B. However, the longitudinal axis of the insertion needle 241 is also parallel to the longitudinal axis of the cannula 203.

In contrast to the embodiment illustrated in Figs. 1A, 1B, 1C and 1D, the medication fluid 233 is introduced into the chamber 243 via the opening 245 or via another opening 246, wherein the opening 245 and the opening 246 are both sealed by a single seal 253. In particular, the seal 253 comprises a continuous material, in particular silicone. As in the embodiment illustrated in Figs. 1A-1D, the cable portion 239 of the sensor system 207 contacts contact pads 265, which either lead to socket elements or are contacted to a transmitter as explained with respect to Fig. 1D. Further, the casing 201 comprises a first casing portion 202 and a second, separate casing portion 204, which can be locked relative to each other using a locking mechanism 206 including a releasable snap connection.

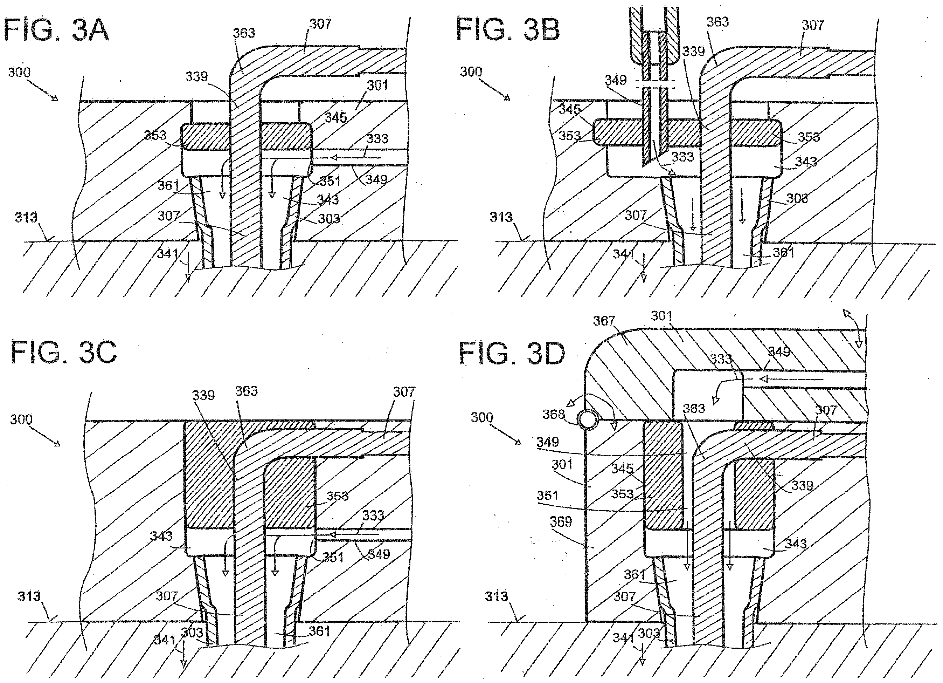

Figs. 3A, 3B, 3C and 3D schematically illustrate cross-sectional side views of medical apparatuses 300 according to other embodiments of the present invention, wherein in Figs. 3A and 3B different options of supplying the medication fluid 333 to the chamber 343 are illustrated. In
Fig. 3A the medication fluid 333 is supplied using a supply channel 349, which communicates with the chamber 343 via a further opening 351 arranged closer to the cannula 303 than the opening 345 through which the cable portion 339 of the sensor system 307 is led to an outside of the casing 301.

In contrast, in Fig. 3B the medication fluid 333 is introduced into the chamber 343 via a supply channel 349 penetrating the seal 353 sealing the chamber 343 against fluid communication via the opening 345.

In Figs. 3C and 3D, different configurations of guiding the medication fluid 333 and guiding the cable portion 339 through the seal 353 are illustrated. In particular, an insertion assembly comprising the cannula 303, the insertion needle 105 and the sensor portion 327 of the sensor 307, are inserted using a (not illustrated) C-shaped (when viewed along an insertion direction, i.e. longitudinal direction of the needle) insertion needle. Thereby, a bent cable portion 339 of the sensor system 307 may be supported, which may have its bent portion or kink 363 arranged within the seal 353, as is illustrated in Fig. 3C.

As is illustrated in Fig. 3D, an opening 351 is arranged in the seal 353, thereby permitting the chamber 343 to be placed in fluid communication with the supply channel 349, which is provided after having withdrawn the insertion needle by turning a portion 367 of the casing 301 relative to another portion 369 of the casing 301 around a turning axis 368 (perpendicular to the drawing page of Fig. 3D). The size of the opening 351 may be larger than the radial extension of the sensor 307, but may equal to or may be smaller than the radial extension of the insertion needle.

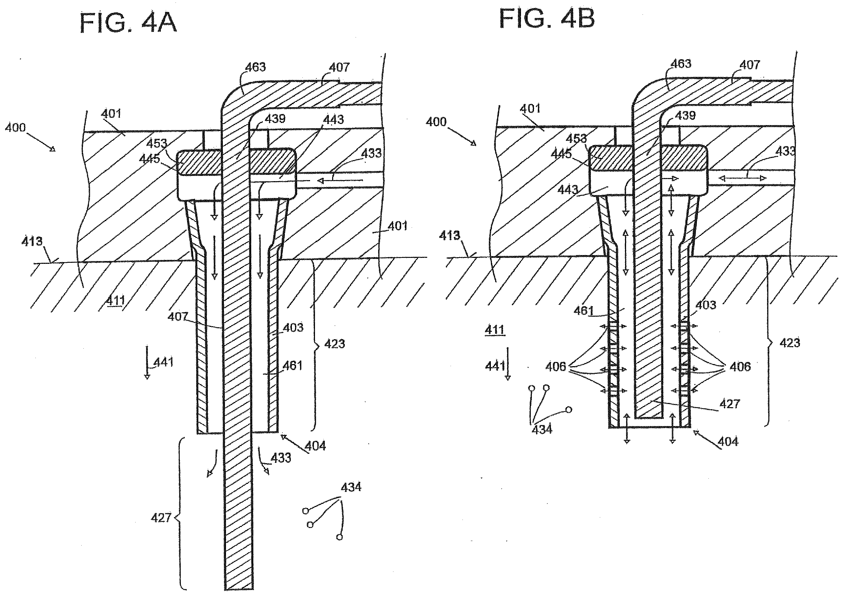

Fig. 4 schematically illustrates portions of medical apparatuses 400 according to embodiments of the present invention, wherein emphasis is laid on the configuration of the sensor system, in particular the detection portion of the sensor system. The different configurations for the sensor system, in particular the detection portion of the sensor system, may be applied to any of the afore mentioned and described embodiments, such as those illustrated in Figs. 1A-1D, 2A, 2B and 2C and Figs. 3A, 3B, 3C and 3D.

Fig. 4 illustrates a portion of a medical apparatus 400, wherein the insertion needle has already been withdrawn, such that the cannula 403 and a portion of the sensor system 407, in particular a detection portion 427, are inserted into the tissue 411 below the surface 413 of the organism. As can be observed from Fig. 4A, the detection portion 427 being responsive to the substance 434 to be detected, in particular glucose, protrudes beyond the longitudinal end 404 of the cannula 403, wherein at the longitudinal end or exit port 404, the medication fluid 433 exits the lumen 461 of the cannula 403. The detection portion 427 may protrude by an amount between 0.1 mm-10 mm depending on the application.

In Fig. 4B an alternative embodiment of the medical apparatus 400 is illustrated, wherein a situation is shown analogous to the situation illustrated in Fig. 4A, wherein the cannula 403 is inserted into the tissue 411, wherein the detection portion 427 is located within the lumen 461 of the cannula 403. In this case, the cannula 403 is a perforated cannula having plural exit ports 406 through which on one hand the medication fluid 433 can exit the lumen 461 of the cannula 403 and through which on the other hand the substance 434, in particular glucose, within the tissue can enter the lumen 461 of the cannula 403 to get in contact with the detection portion 427 of the sensor system 407. Thereby, even in this case, the medical apparatus 400 is enabled to detect the substance, in particular to determine the concentration of the substance within the tissue and to derive therefrom the concentration of the substance within the blood. In particular, the substance 434 to be detected may be sucked into the lumen of the cannula 403 by reversing a flow direction of a pump from a medication fluid supply mode to a substance detection mode.

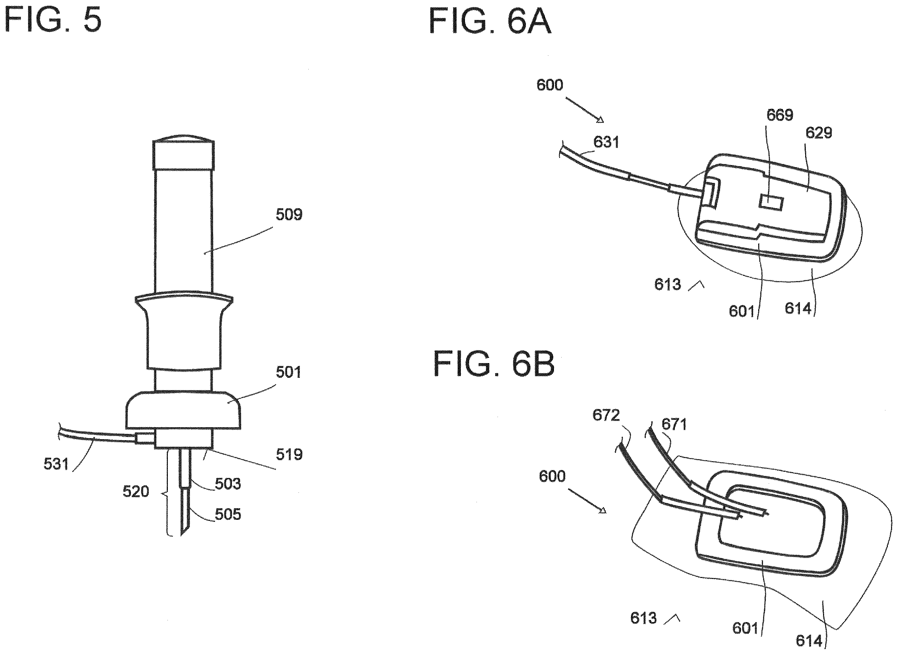

Fig. 5 schematically illustrates a medical apparatus 500 in a perspective view having a gripping portion 509 releasably attached to the casing 501. The casing comprises at the surface 519 an adhesive to attach to a skin surface of a patient, when the insertion assembly 520 comprising a cannula 503, an insertion needle 505 and a (not illustrated) sensor portion is inserted into tissue of a patient. After inserting the insertion assembly 520, the gripping portion 509 may be removed from the casing 501.

Figs. 6A and 6B illustrate perspective views of embodiments of a medical apparatus 600 according to the present invention. Fig. 6A illustrates a casing 601 being attached to skin 613 using an adhesive tape 614. A medication fluid supply tube 631 supplies insulin via a connector 647 to a chamber within the casing 601. From there, the insulin is supplied using a cannula into the fat tissue of the patient. At the casing 601 an adapter 629 comprising a transmitter 669 (such as illustrated in Fig. 1D) is attached. Via the transmitter 669 within the adapter 629, the sensor signals may be transmitted to a monitoring or control system.

Fig. 6B illustrates another embodiment showing a perspective view of a medical apparatus 600 attached via the adhesive tape 614 to the skin 613 of the patient, wherein the casing 601 is adhered to the adhesive tape 614 at the other side. Instead of using a transmitter as in Fig. 6A, the electrical sensor signals are carried away using cables 671 and 672 being connected to two different electrodes of the detection portion of the sensor system inserted into the tissue of the patient.

The casing may be made out of materials such as polyethylene, polyurethane, polycarbonate, polyvinyl chloride, polypropylene, ceramics, composites, or the like.

## Claims

1. Medical apparatus for supplying a medication fluid into an organism and for detecting a substance of the organism, the medical apparatus comprising:
a casing (101, 201, 301, 401, 501, 601) having a chamber (143, 243, 343, 443) for accommodating the medication fluid (133, 233, 333, 433) and having an opening (145, 245, 246, 345, 445) in fluid communication with the chamber;
a cannula (103, 203, 303, 403) having a lumen (161, 261, 361, 461) being in fluid communication with the chamber;
an insertion needle (105, 205) having a receptacle, wherein at least a portion of the insertion needle is removably arrangeable within the lumen of the cannula;
a sensor system (107, 207, 307) for detecting the substance of the organism, wherein the receptacle of the insertion needle is configured to removably at least partially receive the sensor system;
**characterised by**
a seal (153, 253, 353, 453),
wherein the seal is adapted to annularly sealingly surround the insertion needle, when the insertion needle is arranged in the opening (145, 245, 246, 345, 445),
wherein the seal is adapted to annularly sealingly surround a portion (139, 239, 339, 439) of the sensor system, when the insertion needle is removed from the opening and the portion (139, 239, 339, 439) of the sensor system is arranged in the opening (145, 245, 246, 345, 445),
wherein the seal is adapted to seal the chamber from fluid communication via the opening (145, 245, 246, 345, 445) when the insertion needle is arranged in the opening,
wherein the seal is adapted to seal the chamber from fluid communication via the opening (145, 245, 246, 345, 445) when the insertion needle is removed from the opening and a portion (139, 239, 339, 439) of the sensor system is arranged in the opening.

2. Medical apparatus according to claim 1, wherein the cannula (103, 203, 303, 403) is flexible and comprises an insertion portion (123, 223) protruding from the casing (101, 201, 301, 401, 501, 601) away from the chamber.

3. Medical apparatus according to claim 2, wherein the sensor system comprises a detection portion (127, 227) protruding beyond the insertion portion (123, 223, 423) of the cannula away from the chamber, wherein the detection portion is configured to detect the substance.

4. Medical apparatus according to claim 2, wherein the insertion portion of the cannula comprises at least one entry port (406) at an outer surface of the insertion portion (123, 223, 423) of the cannula, the entry port allowing entry of the substance into the lumen of the cannula, the insertion portion in particular being perforated, wherein the sensor system comprises a detection portion arranged within the insertion portion of the cannula, wherein the detection portion is configured to detect the substance entered into the lumen of the cannula.

5. Medical apparatus according to one of the preceding claims,
wherein an insertion assembly is insertable into the organism, the insertion assembly comprising at least a portion of each of the cannula, the insertion needle and the sensor system,
the medical apparatus further comprising:
a placement facility having a body and a lever, the lever being removably connectable with the casing, wherein moving the lever enables moving the casing in a direction along the longitudinal axis of the insertion needle relative to a body of the placement facility for inserting the insertion assembly into the organism.

6. Medical apparatus according to one of the four preceding claims, wherein an infusion and monitoring assembly is obtained by withdrawing the insertion needle from the insertion assembly, wherein using the infusion and monitoring assembly the medication fluid can be supplied to the organism and the substance can be detected simultaneously.

7. Medical apparatus according to the preceding claim, wherein the medication fluid guided inside the lumen of the cannula is in contact with a detection portion (127, 227) of the sensor system when the medication fluid is supplied into the organism.

8. Medical apparatus according to one of the preceding claims, wherein the cannula has a tubular shape having a cross-sectional inner extent which matches a cross-sectional outer extent of the insertion needle,
wherein the casing has a further opening (151, 351) in fluid communication with the chamber.

9. Medical apparatus according to one of the preceding claims, wherein the casing has a connector (147, 247, 647) connecting a medication fluid tube (131, 231, 631) for supplying medication fluid to the chamber, wherein the connector is in particular provided at the further opening (151, 351).

10. Medical apparatus according to claim 9, wherein the further opening (351) has fluid communication with the chamber (343), wherein the connector is provided at the further opening (351), and wherein the further opening in particular has a cross-sectional size which is larger than a cross-sectional size of the sensor but smaller than a cross-sectional size of the insertion needle.

11. Medical apparatus according to one of the preceding claims, further comprising an adapter (129, 629) having at least one electrical terminal, wherein the adapter is releasably connectabe to the casing and wherein the electrical terminal is electrically contactable to a sensor cable of the sensor system.

12. Medical apparatus according to the preceding claim, wherein the adapter comprises a transmitter (169, 669) for transmitting sensor signals received from the sensor system.

13. Medical apparatus according to one of the preceding claims, wherein the sensor system comprises a kink (163, 363, 463), in particular at the opening or at a location farther away from the chamber than the opening.

14. Medical apparatus according to one of the preceding claims, wherein the sensor system is at least partially guided within the insertion needle within the cannula through the seal when inserting the insertion assembly into the tissue by moving the insertion needle into the tissue,
the medical apparatus further comprising a pump system connectable to the medication fluid tube in fluid communication with the chamber for supplying the medication fluid into the chamber and from there via the cannula into the organism.

15. Medical apparatus according to the preceding claim, further comprising a controller communicatively, in particular electrically, connected to the sensor system and electrically connected to the pump system, wherein the controller is adaped to control the pump system based on a signal received from the sensor system.

## Patentansprüche

1. Medizinische Vorrichtung zum Liefern eines Medikationsfluides in einen Organismus und zum Detektieren einer Substanz von dem Organismus, wobei die medizinische Vorrichtung aufweist:
ein Gehäuse (101, 201, 301, 401, 501, 601), mit einer Kammer (143, 243, 343, 443) zum Aufnehmen des Medikationsfluides (133, 233, 333, 433) und einer Öffnung (145, 245, 246, 345, 445), welche in fluidischer Kommunikation mit der Kammer ist;
eine Kanüle (103, 203, 303, 403), welche ein Lumen (161, 261, 361, 461) hat, welches in fluidischer Kommunikation mit der Kammer ist;
eine Einführnadel (105, 205), welche einen Behälter hat, wobei zumindest ein Teil der Einführnadel entfernbar innerhalb des Lumens der Kanüle angeordnet ist;
ein Sensorsystem (107, 207, 307) zum Detektieren der Substanz von dem Organismus, wobei der Behälter von der Einführnadel konfiguriert ist, zum entfernbar zumindest teilweisen Empfangen des Sensorsystems; **gekennzeichnet, durch**
eine Dichtung (153, 253, 353, 453),
wobei die Dichtung eingerichtet ist, die Einführnadel ringförmig abdichtend zu umschließen, wenn die Einführnadel in der Öffnung (145, 245, 246, 345, 445) angeordnet ist,
wobei die Dichtung eingerichtet ist, um einen Teil (139, 239, 339, 439) von dem Sensorsystems ringförmig abdichtend zu umschließen, wenn die Einführnadel von der Öffnung entfernt ist und der Teil (139, 239, 339, 439) von dem Sensorsystem in der Öffnung (145, 245, 246, 345, 445) angeordnet ist,
wobei die Dichtung eingerichtet ist, um die Kammer von der fluidischen Kommunikation via der Öffnung (145, 245, 246, 345, 445) abzudichten, wenn die Einführnadel in der Öffnung angeordnet ist,
wobei die Dichtung eigerichtet ist, um die Kammer von der fluidischen Kommunikation via der Öffnung (145, 245, 246, 345, 445) abzudichten, wenn die Einführnadel von der Öffnung entfernt ist und ein Teil (139, 239, 339, 439) von dem Sensorsystem in der Öffnung angeordnet ist.

2. Medizinische Vorrichtung gemäß Anspruch 1, wobei die Kanüle (103, 203, 303, 403) flexibel ist und einen Einführteil (123, 223) aufweist, welcher von dem Gehäuse (101, 201, 301, 401, 501, 601) weg von der Kammer herausragt.

3. Medizinische Vorrichtung gemäß Anspruch 2, wobei das Sensorsystem einen Detektionsteil (127, 227) aufweist, welcher über den Einführteil (123, 223, 423) von der Kanüle weg von der Kammer herausragt, wobei der Detektionsteil konfiguriert ist die Substanz zu detektieren.

4. Medizinische Vorrichtung gemäß Anspruch 2, wobei der, Einführteil von der Kanüle zumindest einen Eingangsport (406) an einer äußeren Oberfläche von dem Einführteil (123, 223, 423) von der Kanüle aufweist, wobei der Eingangsport den Eingang der Substanz in das Lumen von der Kanüle erlaubt, wobei der Einführteil insbesondere perforiert ist, wobei das Sensorsystem einen Detektionsteil aufweist, welcher innerhalb des Einführteils von der Kanüle angeordnet ist, wobei der Detektionsteil konfiguriert ist, um die Substanz, welche in das Lumen von der Kanüle eingedrungen ist, zu detektieren.

5. Medizinische Vorrichtung gemäß einem der vorhergehenden Ansprüche, wobei eine Einführanordnung in den Organismus einführbar ist, wobei die Einführanordnung zumindest einen Teil aufweist von jedem von der Kanüle, der Einführnadel und dem Sensorsystem,
wobei die medizinische Vorrichtung ferner aufweist:
eine Platziereinrichtung, welche einen Körper und einen Hebel hat, wobei der Hebel entfernbar verbindbar mit dem Gehäuse ist, wobei ein Bewegen des Hebels ein Bewegen des Gehäuses ermöglicht in eine Richtung entlang der longitudinalen Achse von der Einführnadel relativ zu einem Körper von der Platziervorrichtung zum Einführen der Einführanordnung in den Organismus.

6. Medizinische Vorrichtung gemäß einem der vier vorhergehenden Ansprüche, wobei eine Infusions- und Überwachungsanordnung erhalten wird mittels Herausziehens der Einführnadel von der Einführanordnung, wobei mittels der Infusions- und Überwachungsanordnung das Medikationsfluid zu dem Organismus geliefert werden kann und die Substanz simultan detektiert werden kann.

7. Medizinische Vorrichtung gemäß dem vorhergehenden Anspruch, wobei das Medikationsfluid, welches in das Lumen der Kanüle geführt ist, in Kontakt mit einem Detektionsteil (127, 227) von dem Sensorsystem ist, wenn das Medikationsfluid in den Organismus geliefert wird.

8. Medizinische Vorrichtung gemäß einem der vorhergehenden Ansprüche, wobei die Kanüle eine röhrenförmige Form hat, welche eine Querschnitt Innenausdehnung hat, welche mit einer Querschnitt Außenausdehnung von der Einführnadel übereinstimmt,
wobei das Gehäuse eine weitere Öffnung (151, 351) in fluidischer Kommunikation mit der Kammer hat.

9. Medizinische Vorrichtung gemäß einem der vorhergehenden Ansprüche, wobei das Gehäuse einen Konnektor (147, 247, 647) hat, welcher eine Medikationsfluid Röhre (131, 231, 631) verbindet zum Liefern von Medikationsfluid zu der Kammer, wobei der Konnektor insbesondere an der weiteren Öffnung (151, 351) bereitgestellt wird.

10. Medizinische Vorrichtung gemäß Anspruch 9, wobei die weitere Öffnung (351) fluidische Kommunikation mit der Kammer (343) hat, wobei der Konnektor an der weiteren Öffnung (351) bereitgestellt ist und wobei die weitere Öffnung insbesondere eine Querschnittsgröße hat, welche größer als eine Querschnittsgröße von dem Sensor aber kleiner als eine Querschnittsgröße von der Einführnadel ist.

11. Medizinische Vorrichtung gemäß einem der vorhergehenden Ansprüche, ferner aufweisend einen Adapter (129, 629), welcher zumindest einen elektrischen Anschluss hat, wobei der Adapter lösbar mit dem Gehäuse verbindbar ist und wobei der elektrische Anschluss elektrisch mit einem Sensorkabel von dem Sensorsystem verbindbar ist.

12. Medizinische Vorrichtung gemäß dem vorhergehenden Anspruch, wobei der Adapter einen Transmitter (169, 669) zum Übertragen von Sensorsignalen aufweist, welche von dem Sensorsystem empfangen werden.

13. Medizinische Vorrichtung gemäß einem der vorhergehenden Ansprüche, wobei das Sensorsystem aufweist einen Knick (163, 363, 463), insbesondere an der Öffnung oder einem Ort weiter entfernt von der Kammer als die Öffnung.

14. Medizinische Vorrichtung gemäß einem der vorhergehenden Ansprüche, wobei das Sensorsystem zumindest teilweise geführt ist innerhalb der Einführnadel innerhalb der Kanüle durch die Dichtung, wenn die Einführanordnung in das Gewebe eingeführt wird mittels Bewegens der Einführnadel in das Gewebe,
wobei die medizinische Vorrichtung ferner ein Pumpensystem aufweist, welches mit der Medikationsfluid Röhre verbindbar ist, welche in fluidischer Kommunikation mit der Kammer ist zum Liefern des Medikationsfluides in die Kammer und von dort via die Kanüle in den Organismus.

15. Medizinische Vorrichtung gemäß dem vorhergehenden Anspruch, ferner aufweisend einen Controller, welcher kommunikativ, insbesondere elektrisch, mit dem Sensorsystem verbunden ist und elektrisch mit dem Pumpensystem verbunden ist, wobei der Controller eingerichtet ist das Pumpensystem zu steuern basierend auf einem Signal, welches von dem Sensorsystem empfangen wurde.

## Revendications

1. Appareil médical destiné à acheminer un fluide de médication dans un organisme et à détecter une substance de l'organisme, l'appareil médical comprenant :
une enveloppe (101, 201, 301, 401, 501, 601) présentant une chambre (143, 243, 343, 443) pour accueillir le fluide de médication (133, 233, 333, 433) et présentant une ouverture (145, 245, 246, 345, 445) en communication fluidique avec la chambre ;
une canule (103, 203, 303, 403) présentant une lumière (161, 261, 361, 461) en communication fluidique avec la chambre ;
une aiguille d'insertion (105, 205) présentant un réceptacle, dans lequel au moins une portion de l'aiguille d'insertion peut être agencée de manière amovible dans la lumière de la canule ;
un système de capteur (107, 207, 307) destiné à détecter la substance de l'organisme, dans lequel le réceptacle de l'aiguille d'insertion est configuré pour recevoir de manière amovible, au moins partiellement, le système de capteur ;
**caractérisé par**
un joint (153, 253, 353, 453),
dans lequel le joint est adapté pour entourer de manière annulaire et étanche l'aiguille d'insertion, quand l'aiguille d'insertion est agencée dans l'ouverture (145, 245, 246, 345, 445),
dans lequel le joint est adapté pour entourer de manière annulaire et étanche une portion (139, 239, 339, 439) du système de capteur, quand l'aiguille d'insertion est retirée de l'ouverture et la portion (139, 239, 339, 439) du système de capteur est agencée dans l'ouverture (145, 245, 246, 345, 445),
dans lequel le joint est adapté pour rendre la chambre étanche à la communication fluidique par le biais de l'ouverture (145, 245, 246, 345, 445) quand l'aiguille d'insertion est agencée dans l'ouverture,
dans lequel le joint est adapté pour rendre la chambre étanche à la communication fluidique par le biais de l'ouverture (145, 245, 246, 345, 445) quand l'aiguille d'insertion est retirée de l'ouverture et une portion (139, 239, 339, 439) du système de capteur est agencée dans l'ouverture.

2. Appareil médical selon la revendication 1, dans lequel la canule (103, 203, 303, 403) est souple et comprend une portion d'insertion (123, 223) faisant saillie à partir de l'enveloppe (101, 201, 301, 401, 501, 601) loin de la chambre.

3. Appareil médical selon la revendication 2, dans lequel le système de capteur comprend une portion de détection (127, 227) faisant saillie au-delà de la portion d'insertion (123, 223, 423) de la canule loin de la chambre, dans lequel la portion de détection est configurée pour détecter la substance.

4. Appareil médical selon la revendication 2, dans lequel la portion d'insertion de la canule comprend au moins un orifice d'entrée (406) au niveau d'une surface-extérieure de la portion d'insertion (123, 223, 423) de la canule, l'orifice d'entrée permettant l'entrée de la substance dans la lumière de la canule, la portion d'insertion étant en particulier perforée, dans lequel le système de capteur comprend une portion de détection agencée à l'intérieur de la portion d'insertion de la canule, dans lequel la portion de détection est configurée pour détecter la substance entrée dans la lumière de la canule.

5. Appareil médical selon l'une des revendications précédentes, dans lequel un ensemble d'insertion peut être inséré dans l'organisme, l'ensemble d'insertion comprenant au moins une portion de chacun parmi la canule, l'aiguille d'insertion et le système de capteur,
l'appareil médical comprenant en outre :
une installation de mise en place présentant un corps et un levier, le levier pouvant être connecté de manière amovible à l'enveloppe, dans lequel le déplacement du levier permet le déplacement de l'enveloppe dans une direction le long de l'axe longitudinal de l'aiguille d'insertion par rapport à un corps de l'installation de mise en place pour insérer l'ensemble d'insertion dans l'organisme.

6. Appareil médical selon l'une des quatre revendications précédentes, dans lequel un ensemble de perfusion et de contrôle est obtenu en retirant l'aiguille d'insertion de l'ensemble d'insertion, dans lequel en utilisant l'ensemble de perfusion et de contrôle, le fluide de médication peut être acheminé jusqu'à l'organisme et la substance peut être détectée simultanément.

7. Appareil médical selon la revendication précédente, dans lequel le fluide de médication guidé à l'intérieur de la lumière de la canule est en contact avec une portion de détection (127, 227) du système de capteur quand le fluide de médication est acheminé dans l'organisme.

8. Appareil médical selon l'une des revendications précédentes, dans lequel la canule présente une forme tubulaire présentant une étendue intérieure en coupe transversale qui correspond à une étendue extérieure en coupe transversale de l'aiguille d'insertion,
dans lequel l'enveloppe présente une ouverture supplémentaire (151, 351) en communication fluidique avec la chambre.

9. Appareil médical selon l'une des revendications précédentes, dans lequel l'enveloppe présente un connecteur (147, 247, 647) connectant un tube de fluide de médication (131, 231, 631) pour acheminer le fluide de médication jusqu'à la chambre, dans lequel le connecteur est en particulier disposé au niveau de l'ouverture supplémentaire (151, 351).

10. Appareil médical selon la revendication 9, dans lequel l'ouverture supplémentaire (351) présente une communication fluidique avec la chambre (343), dans lequel le connecteur est disposé au niveau de l'ouverture supplémentaire (351), et dans lequel l'ouverture supplémentaire présente en particulier une taille en coupe transversale qui est supérieure à une taille en coupe transversale du capteur mais inférieure à une taille en coupe transversale de l'aiguille d'insertion.

11. Appareil médical selon l'une des revendications précédentes, comprenant en outre un adaptateur (129, 629) présentant au moins une borne électrique, dans lequel l'adaptateur peut être connecté de manière libérable à l'enveloppe et dans lequel la borne électrique peut être électriquement connectée à un câble de capteur du système de capteur.

12. Appareil médical selon la revendication précédente, dans lequel l'adaptateur comprend un émetteur (169, 669) destiné à transmettre des signaux de capteur reçus en provenance du système de capteur.

13. Appareil médical selon l'une des revendications précédentes, dans lequel le système de capteur comprend un coude (163, 363, 463), en particulier au niveau de l'ouverture ou à un endroit plus éloigné de la chambre que l'ouverture.

14. Appareil médical selon l'une des revendications précédentes, dans lequel le système de capteur est au moins partiellement guidé dans l'aiguille d'insertion dans la canule à travers le joint lors de l'insertion de l'ensemble d'insertion dans le tissu par le déplacement de l'aiguille d'insertion dans le tissu,
l'appareil médical comprenant en outre un système de pompe pouvant être connecté au tube de fluide de médication en communication fluidique avec la chambre pour acheminer le fluide de médication dans la chambre et de là par le biais de la canule dans l'organisme.

15. Appareil médical selon la revendication précédente, comprenant en outre un dispositif de commande connecté de manière communicative, en particulier électrique, au système de capteur et électriquement connecté au système de pompe, dans lequel le dispositif de commande est adapté pour commander le système de pompe sur la base d'un signal reçu en provenance du système de capteur.
